# EUROPEAN PATENT SPECIFICATION

(11) **EP 2 203 451 B1**
(45) Date of publication and mention of the grant of the patent: **30.07.2014**
(21) Application number: 08836001.1
(22) Date of filing: 03.10.2008
(51) Int. Cl.: C07D 473/34, A61K 31/52, A01N 43/00

(54) **SUBSTITUTED 6-(ALKYLBENZYLAMINO)PURINE DERIVATIVES FOR USE AS CYTOKININ RECEPTOR ANTAGONISTS AND PREPARATIONS CONTAINING THESE DERIVATIVES**
SUBSTITUIERTE 6-(ALKYLBENZYLAMINO)PURIN-DERIVATE ALS ZYTOKININ-REZEPTORANTAGONISTEN UND ZUBEREITUNGEN MIT DIESEN DERIVATEN
DÉRIVÉS DE 6-(ALKYLBENZYLAMINI)PURINE SUBSTITUÉS S'UTILISANT COMME ANTAGONISTES DU RÉCEPTEUR DE LA CYTOKINE ET PRÉPARATIONS CONTENANT CES DÉRIVÉS

(30) Priority: 05.10.2007 CZ 20070691
(43) Date of publication of application: 07.07.2010
(73) Proprietor: Univerzita palackeho V Olomouci, 77147 Olomouc (CZ); Freie Universität Berlin, 14195 Berlin (DE)
(72) Inventor: SPICHAL, Lukas, 779 00 Olomouc (CZ); POPA, Igor, 779 00 Olomouc (CZ); VOLLER, Jiri, 635 00 Brno-Bystrc (CZ); DOLEZAL, Karel, 78365 Hlubocky (CZ); STRNAD, Miroslav, 779 00 Olomouc (CZ); WERNER, Tomas, 10829 Berlin (DE); SCHMULLING, Thomas, 14052 Berlin (DE)
(74) Representative: Gabrielova, Marta
(86) International application number: PCT/CZ2008/000118
(87) International publication number: WO 2009/043320

(56) References cited:
- WO-A-97/20842
- WO-A-03/040144
- BRATHE A ET AL: "Synthesis of 6-Alkenyl- and 6-Alkynylpurines with Cytokinin Activity" TETRAHEDRON, ELSEVIER SCIENCE PUBLISHERS, AMSTERDAM, NL, vol. 55, no. 1, 1 January 1999 (1999-01-01), pages 211-228, XP004150864 ISSN: 0040-4020
- RIEFLER M ET AL: "Arabidopsis cytokinin receptor mutants reveal functions in shoot growth, leaf senescence, seed size, germination, root development, and cytokinin metabolism", THE PLANT CELL, AMERICAN SOCIETY OF PLANT BIOLOGISTS, US, vol. 18, 1 January 2006 (2006-01-01), pages 40-54, XP002362525, ISSN: 1040-4651, DOI: 10.1105/TPC.105.037796
- HIGUCHI MASAYUKI ET AL: "In planta functions of the Arabidopsis cytokinin receptor family", PROCEEDINGS OF THE NATIONAL ACADEMY OF SCIENCES, NATIONAL ACADEMY OF SCIENCES, US, vol. 101, no. 23, 8 June 2004 (2004-06-08) , pages 8821-8826, XP002362523, ISSN: 0027-8424, DOI: 10.1073/PNAS.0402887101
- NISHIMURA ET AL.: "Genetic analysis of arabidopsis histidine kinase genes encoding cytokinin receptors reveals their overlapping biological functions in the regulation of shoot and root growth in arabidopsis thaliana", THE PLANT CELL, 2004, pages 1-13,

## Description

### Technical Field

The invention relates to 6-(alkylbenzylamino)purine derivatives, their use as cytokinin receptor antagonists and preparations containing these derivatives.

### Background Art

Cytokinins are plant hormones that play essential roles in the regulation of various aspects of plant growth and development. They include variety of chemicals with different degrees of structural similarity, some of which occur naturally in plants, while others were prepared synthetically (Mok & Mok Ami. Rev. Plant Physiol. Plant Mol. Biol. 52: 89-118, 2001). The natural cytokinins are adenine derivatives that can be classified according to the nature of their N⁶-side chain as either isoprenoid or aromatic cytokinins. The important representatives of these two classes are zeatin and 6-benzylaminopurine.

Cytokinins are key regulators of the plant cell cycle and the induction of cell division is considered diagnostic for this class of plant hormones. The molecular basis of this activity is only partially understood and may differ in different cell types. Cytokinins have been found to control tyrosine dephosphorylation and activation of p34^{cdc2}-like H1 histone kinase (Zhang et al., Planta 200: 2-12, 1996), as well as the transcriptional activation of *cyclin D3* (Riou-Khamlichi et al. Science 283: 1541-1544, 1999). Some of the important physiological and developmental processes, which are controlled by cytokinin, such as the formation and activity of apical meristems, floral development, the breaking of bud dormancy, and seed germination, are at least in part functionally linked to cell cycle control mechanisms.

How plants recognize cytokinins was for a long time puzzling. Recently, several cytokinin receptors were identified in *Arabidopsis* (Inoue et al. Nature 409: 1060-1063, 2001) and *Zea mays* (Yonekura-Sakakibara et al. Plant Physiol. 134: 1654-1661, 2004). To date, three cytokinin receptors have been identified in *Arabidopsis,* AHK2, AHK3 and CRE1/AHK4. All are membrane-located sensor histidine kinases with extracellular ligand-binding domain and cytoplasmic His-kinase domain. It has been shown that the cytokinin signal is transmitted by a multi-step phospho-relay system that has long been known in prokaryotes and lower eukaryotes. Among higher eukaryotes, the two-component signalling pathways are only found in plants (Ferreira & Kieber, Curr. Opin. Plant Biol. 8: 518-525, 2005). The development of agonists and antagonists with a particular physiological effect is useful in mechanism-of-action studies of biologically active natural products. The design of potential cytokinin antagonists has been based on the assumptions 1) that active cytokinins bind to one or more cellular receptor sites, and 2) that it should be possible to prepare compounds that have minimum cytokinin activity, but retain a high degree of structural similarity to the cytokinins that permits them to compete for available cytokinin receptor sites, thereby diminishing the biological activity of cytokinins. The potent naturally-occurring cytokinin N⁶-isopentenyladenine served as the basis for initial structure-activity studies. Modification of the heterocyclic purine system yielded the first analogues with antagonistic activity that greatly reduced cytokinin activity in bioassays (Skoog & Armstrong, Annu. Rev. Plant Physiol. 21:359-384, 1970; Skoog et al., Phytochemistry 6:1169-1192, 1967). Identification of a class of cytokinin antagonists by rational processes has shown that further structurally similar heterocyclic compounds can possess the cytokinin antagonistic activity. Consequently, a number of substituted pyrrolo[2,3-*d*]pyrimidines, pyrazolo[4,3-*d*]pyrimidines, *s-*triazines, N-benzyl-N'-phenylureas, and N-arylcarbamates were subsequently prepared and tested for their ability to inhibit cytokinin-promoted processes in various bioassays, and a number of them were identified as potential anticytokinins (Hecht, Proc. Natl. Acad. Sci USA 68: 2608-2610, 1971; Skoog et al. Phytochemistry 1, 25-37, 1973; Shimizu et al. J. Agric. Food Chem. 37:236-240, 1989, Iwamura et al. Biochim. Biophys. Res. Commun. 57:412-416, 1974; Hecht et al. 1976: US4,282,361; Skoog et al. 1981: US3,988, 38). Because of their structural similarity to natural cytokinins and because of their antagonistic effects, which were reversible depending on increase of the cytokinin concentration, it was hypothesised that these compounds work through interaction with a common cellular target, *i.e.* the cytokinin receptors (in Mok & Mok, CRC Press, Boca Raton, USA, pp.43-55, 1994). However, until recently, direct proof that cytokinin receptors are the sites of cytokinin-anticytokinin interactions was lacking, because no cytokinin receptors had been identified at that time. Recent discoveries and advances in our understanding of cytokinin signalling motivated us to re-examine anticytokinin modes of action. First, we showed that representative anticytokinins are not competitive inhibitors of two *Arabidopsis* cytokinin receptors. Using mainly the potent anticytokinin 3-methyl-7-pentylaminopyrazolo[4,3-*d*]pyrimidine as a representative example, we also showed that this compound inhibits cell cycle progression and causes cellular changes consistent with responses to known cyclin-dependent kinase (CDK) inhibitors. We demonstrated CDK inhibition by anticytokinins in plants and humans and revealed the binding of ANCYT1 to the ATP-binding pocket of human CDK2 (Spichal et al. J. Biol. Chem. 282:14356-63, 2007).

Perception of cytokinins was recently shown to be the crucial step in the regulation of various aspects of plant growth and development. Study of plant loss-of-function cytokinin receptor mutants and comprehensive analysis of all single, double and triple mutants showed that decreased sensing of cytokinins led to altered shoot growth, retarded leaf senescence, enhancement of seed size, shortened timing of germination and enhancement of root system (Higuchi et al. Proc. Natl. Acad. Sci USA 101: 8821-8826, 2004; Nishimura et al. Plant Cell 16: 1365-1377, 2004; Riefler et al. Plant Cell 18: 40-54, 2006). Loss-of-function and gain-of-function mutations in legume cytokinin receptors revealed direct involvement of cytokinin perception in nodule organogenesis (Gonzalez-Rizzo et al. Plant Cell 18: 2680-2693, 2006; Murray et al. Science 315: 101-104, 2007; Tirichine et al. Science 315: 104-107, 2007). Inhibitor of cytokinin perception - antagonist of cytokinin receptors - which can be applied time- or organ- specifically, could thus find interesting applications in agriculture.

Further reference is made to WO03/040144.

We have recently discovered the first generation of cytokinin receptor antagonists based on substituted 6-(alkylbenzylamino)purines. The most promising substituents in the sense of preparation of specific cytokinin antagonists are 2-hydroxy, 2-amino and 2-nitro groups. Newly prepared compounds show interesting *in vivo* effects on the growth of model plants.

It is an object of this invention to provide cytokinin analogues having unique growth regulating effects and selectivity for cytokinin receptors, that are not toxic for animal cells.

### Disclosure of the Invention

The object of this invention are substituted 6-(alkylbenzylamino)purine derivatives of the general formula I 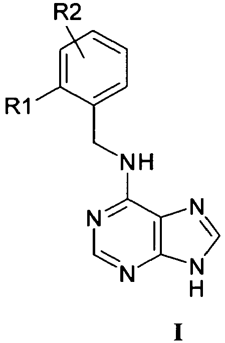 wherein
R1 denotes substituent independently selected from the group comprising hydroxyl, amino, nitro and thio group,
R2 denotes one to four alkyl groups, same or different,
and salts thereof with alkali metals, ammonium or amines,
in the form of racemates or optically active isomers, as well as their addition salts with acids
for use as cytokinin receptor antagonists.

The generic substituent groups have meanings defined in this legend, wherein
amino denotes the group -NH₂,
nitro denotes the group -NO₂,
thio denotes the group -SH,
hydroxy denotes the group -OH,
alkyl denotes branched or unbranched alkyl group containing 1 to 5 carbon atoms.

In the preferred embodiment, the substituted 6-(alkylbenzylamino)purine derivatives of the general formula **I** are: 6-(2-amino-3-methylbenzylamino)purine, 6-(2-amino-4-methylbenzylamino)purine, 6-(2-amino-5-methylbenzylamino)purine, 6-(2-amino-3-ethylbenzylamino)purine, 6-(2-amino-5-ethylbenzylamino)purine, 6-(2-amino-3-isopropylbenzylamino)purine, 6-(2-amino-5-isopropylbenzylamino)purine, 6-(2-hydroxy-3-methylbenzylamino)purine, 6-(2-hydroxy-4-methylbenzylamino)purine, 6-(2-hydroxy-5-methylbenzylamino)purine, 6-(2-hydroxy-6-methylbenzylamino)purine, 6-(2-hydroxy-3-ethylbenzylamino)purine, 6-(2-hydroxy-4-ethylbenzylamino)purine, 6-(2-hydroxy-5-ethylbenzylamino)purine, 6-(2-hydroxy-6-ethylbenzylamino)purine, 6-(2-hydroxy-3-isopropylbenzylamino)purine, 6-(2-hydroxy-5-isopropylbenzylamino)purine, 6-(2-nitro-3-methylbenzylamino)purine, 6-(2-nitro-4-methylbenzylamino)purine, 6-(2-thio-3-methylbenzylamino)purine, 6-(2-thio-5-methylbenzylamino)purine, 6-(2-thio-3-ethylbenzylamino)purine 6-(2-hydroxy-3,5-dimethylbenzylamino)purine, 6-(2-hydroxy-3,4-dimethylbenzylamino)purine, 6-(2-hydroxy-3,6-dimethylbenzylamino)purine, 6-(2-hydroxy-3,4,5-trimethylbenzylamino)purine, 6-(2-amino-3,5-dimethylbenzylamino)purine, 6-(2-amino-3,6-dimethylbenzylamino)purine, 6-(2-hydroxy-3,5-diethylbenzylamino)purine, 6-(2-hydroxy-3,6-diethylbenzylamino)purine
and salts thereof with alkali metals, ammonium or amines,
in the form of racemates or optically active isomers, as well as their addition salts with acids
for use as cytokinin receptor antagonists.

The following substituted 6-(alkylbenzylamino)purine derivatives are particularly preferred, namely: 6-(2-hydroxy-3-methylbenzylamino)purine and 6-(2-hydroxy-5-methylbenzylamino)purine and salts thereof with alkali metals, ammonium or amines, in the form of racemates or optically active isomers, as well as their addition salts with acids for use as cytokinin receptor antagonists.

A further aspect of the invention are the substituted 6-(alkylbenzylamino)purine derivatives of the general formula **I** or salts thereof with alkali metals, ammonium or amines, in the form of racemates or optically active isomers, as well as their addition salts with acids, and their use as cytokinin receptor antagonists for morphogenetic effects leading to the increase of plant root system.

Yet another aspect of the invention are the substituted 6-(alkylbenzylamino)purine derivatives of the general formula **I** or salts thereof with alkali metals, ammonium or amines, in the form of racemates or optically active isomers, as well as their addition salts with acids, and their use as cytokinin receptor antagonists for dispatching grain filling and increase of grain and fruit size of plants and fungi and for shortening of plant seed germination period while leading to the increase of plant root system.

A further aspect of the invention are the substituted 6-(alkylbenzylamino)purine derivatives of the general formula **I** or salts thereof with alkali metals, ammonium or amines, in the form of racemates or optically active isomers, as well as their addition salts with acids, and their use as cytokinin receptor antagonists, especially for increasing of yield and quality of agricultural products while leading to the increase of plant root system.

This invention further concerns substituted 6-(alkylbenzylamino)purine derivatives of the general formula **I,** or salts thereof with alkali metals, ammonium or amines, in the form of racemates or optically active isomers, as well as their addition salts with acids, and their use as cytokinin receptor antagonists, in the production of crops, in particular cereals (wheat, barley, rice, maize, rye, oat, sorghum, and related species), beet (sugar beet and fodded beet); pomes, drupes and soft fruits (apples, pears, plums, peaches, almonds, cherries, strawberries, raspberries and blackberries); leguminous plants (beans, lentils, peas, soybeans); oil plants (rape, mustard, poppy, olives, sunflowers, coconut, *Ricinus,* cocoa beans, groundnuts); cucumber plants (pumpkins, cucumbers, melons); fibre plants (cotton, flax, hemp, jute); citrus fruit (oranges, lemons, grapefruits, mandarins); vegetables (spinach, cinnamon, camphor) or plants such as tobacco, nuts, eggplants, sugar cane, tea, vine grapes, hops, bananas and natural rubber and medicinal plants, as well as ornamentals, while leading to the increase of plant root system. Crops include those which have been rendered tolerant towards classes of growth factors by conventional breeding methods or genetic engineering methods.

The compounds of the general formula **I** are used in unmodified form or, preferably, together with the auxiliary substances (adjuvants) conventionally employed in the art of formulation. To this end they are conventiently formulated as concentrates of active compounds as well as suspensions and dispersions, preferably isotonic water solutions, suspensions and dispersions, diluted emulsions, soluble powders, dusts, granulates, creams, gels, oil suspensions and also encapsulations, e.g. polymeric substances. As with the type of the compositions, the methods of application, such as spraying, atomizing, dusting, scattering, coating or pouring, are chosen in accordance with the intended objectives and the prevailing circumstances. The compositions may be sterilized or contain further adjuvants of a neutral nature such as preservatives, stabilizers, wetting agents or emulgators, solubilizing agents, fertilizers, micronutrinet donors or other formulations for obtaining special effects.

The compounds of the formula **I** can be mixed with other growth regulators, resulting in synergistic activities.

### PREPARATIONS

The preparations comprising the compounds of formula **I** (active ingredients) or salts thereof and, where appropriate, one or more solid or liquid formulation auxiliary substances (adjuvants), are prepared in a manner known *per se,* e.g. by intimately mixing or grinding the active ingredients with the formulation adjuvants, e.g. solvents or solid carriers. In addition, surface-active compounds (surfactants) may also be added in the preparation of the formulations.
Depending on the nature of the compound of formula **I** to be formulated, suitable surface- active compounds are non-ionic, cationic and/or anionic surfactants and surfactant mixtures having good emulsifying, dispersing and wetting properties. Examples of suitable anionic, non-ionic and cationic surfactants are listed, for example, in WO 97/34485.

Also suitable in the preparation compositions containg cytokinin receptor antagonists derived from substituted 6-(alkylbenzylamino)purine derivatives according to the invention are the surfactants conventionally used in formulation technology, which are described, *inter alia,* in "McCutcheon's Detergents and Emulsifiers Annual" MC Publishing Corp., Ridgewood New Jersey, 1981; Stache, H., "Tensid-Taschenbuch", Carl Hanser Verlag, Munich, 1981; and M. and J.Ash, "Encyclopedia of Surfactants", Vol.1-3, Chemical Publishing Co., New York, 1980-81.

The formulation of the preparations with cytokinin receptor antagonists contains from 0.1 to 99 % (w/w), especially from 0.1 to 95 % (w/w), of active ingredient or active ingredient mixture comprising compounds of formula **I,** whereas it contains from 5 to 99.9% of a solid or liquid formulation adjuvants or pharmaceutical carriers, depending on the method of application, and from 0.1 to 25 % (w/w) of a surfactant.

Whereas commercial products are usually formulated as concentrates, the end user will normally employ diluted formulations. The compositions may also comprise further ingredients, such as stabilisers, e.g. vegetable oils or epoxidised vegetable oils (epoxidised coconut 0;1, rapeseed oil or olive oil), antifoams, e.g. silicone oil, preservatives, stabilizers, wetting agents or emulgators, viscosity factors, binders, tackifiers, and also fertilisers or other active ingredients. Preferred formulations have especially the following compositions: (% = percent by weight)

### Emulsifiable concentrates:

| | |
|---|---|
| active ingredient mixture: | 1 to 90 %, preferably 5 to 20 % |
| surfactant: | 1 to 30 %, preferably 10 to 20 % |
| liquid carrier: | 5 to 94 %, preferably 60 to 85 % |

### Dusts:

| | |
|---|---|
| active ingredient mixture: | 0.1 to 10 %, preferably 0.1 to 5 % |
| solid carrier: | 99.9 to 90 %, preferably 99.9 to 95 % |

### Suspension concentrates:

| | |
|---|---|
| active ingredient mixture: | 5 to 75 %, preferably 10 to 50 % |
| water: | 94 to 24 %, preferably 88 to 30 % |
| surfactant: | 1 to 40 %, preferably 2 to 30 % |

### Wettable powders:

| | |
|---|---|
| active ingredient mixture: | 0.5 to 90 %, preferably 1 to 80 % |
| surfactant: | 0.5 to 20 %, preferably 1 to 15 % |
| solid carrier: | 5 to 95 %, preferably 15 to 90 % |

### Granules:

| | |
|---|---|
| active ingredient mixture: | 0.1 to 30 %, preferably 0.1 to 15 % |
| solid carrier: | 99.9 to 70 %, preferably 99.9 to 85 % |

The compositions may also comprise further ingredients, such as stabilisers, e.g. vegetable oils or epoxidised vegetable oils (epoxidised coconut oil, rapeseed oil or soybean oil), anti-foams, e.g. silicone oil, preservatives, viscosity regulators, binders, tackifiers, and also fertilisers or other active ingredients. For the use of cytokinin oxidase inhibitors of the formula **I,** or of compositions comprising them, in the protection of crop plants against the damaging effects of growth regulators, various methods and techniques come into consideration, such as, for example, the following:
i) Seed dressing
   a) Dressing of the seeds with a wettable powder formulation of a compound of the general formula **I** or salt thereof by shaking in a vessel until uniformly distributed over the seed surface (dry dressing). In that procedure approximately from 1 to 500 g of compound of the general formula **I** or salt thereof (4 g to 2 kg of wettable powder) are used per 100 kg of seed.
   b) Dressing of the seeds with an emulsifiable concentrate of a compound of formula **I** or salt thereof according to method a) (wet dressing).
   c) Dressing by immersing the seeds for 1 to 72 hours in a liquor comprising from 100 to 1000 ppm of a compound of formula **I** or salt thereof and preferably subsequently drying the seeds (immersion dressing).

   Dressing the seeds or treating the germinated seedlings are naturally the preferred methods of application, because treatment with the active ingredients is directed entirely at the target crop. Generally, the compounds of general formula **I** or their salts are used in the amount of 1 to 1000 g, preferably from 5 to 250 g, per 100 kg of seeds, but depending on the methodology, which also enables the addition of other active ingredients or micronutrients, the concentration limits indicated can be varied up or down (repeat dressing).
ii) Application as a tank mixture
   A liquid formulation of a mixture of growth regulator and antidote is used, in the ratio by weight of the one to the other from 10:1 to 1 :100, the rate of application of growth regulator being from 0.005 to 5.0 kg per hectare. Such tank mixtures are applied before or after sowing.
iii) Application to the seed furrow
   The compound of formula **I** or salt thereof is introduced into the open, sown seed furrow in the form of an emulsifiable concentrate, wettable powder or granules. Once the seed furrow has been covered over, the growth regulator is applied in the usual manner in the pre-emergence process.
iv) Controlled release of active ingredient
   The compounds of formula **I** or salts thereof are applied in solution to mineral granule carriers or polymerised granules (urea/formaldehyde) and dried. If desired, it is also possible to apply a coating that allows the active ingredient to be released in metered amounts over a specific period of time (coated granules).

### Brief description of drawings

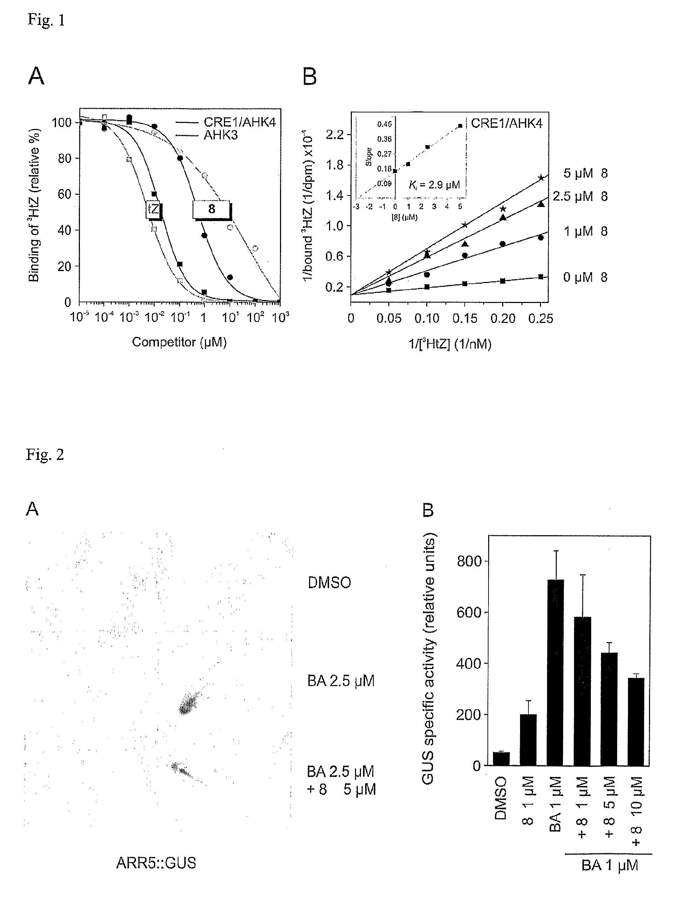

Figure 1 shows binding assay with CRE1/AHK4- and AHK3- containing *E. coli* membranes. (A) Binding of 2 nM 3HtZ was assayed together with increasing concentration of competitor 8, or unlabeled *trans*-zeatin (tZ; positive control). (B) Double-reciprocal plot showing competitive character of inhibition of binding of 3HtZ to the CRE1/AHK4 receptor by compound 8.
Figure 2 shows effect of compound 8 on cytokinin-induced expression of gene *P_{ARR5}::GUS.* (A) *P_{ARR5}::GUS* transgenic *Arabidopsis* seedlings treated with 2.5 µM 6-benzylaminopurine (BA) in the presence or absence of 5 µM compound 8; DMSO (0.1 %) was tested as solvent control. (B) Quantitative evaluation of inhibitory effect of compound 8 on *P_{ARR5}:: GUS* gene expression triggered by 1 µM BA.

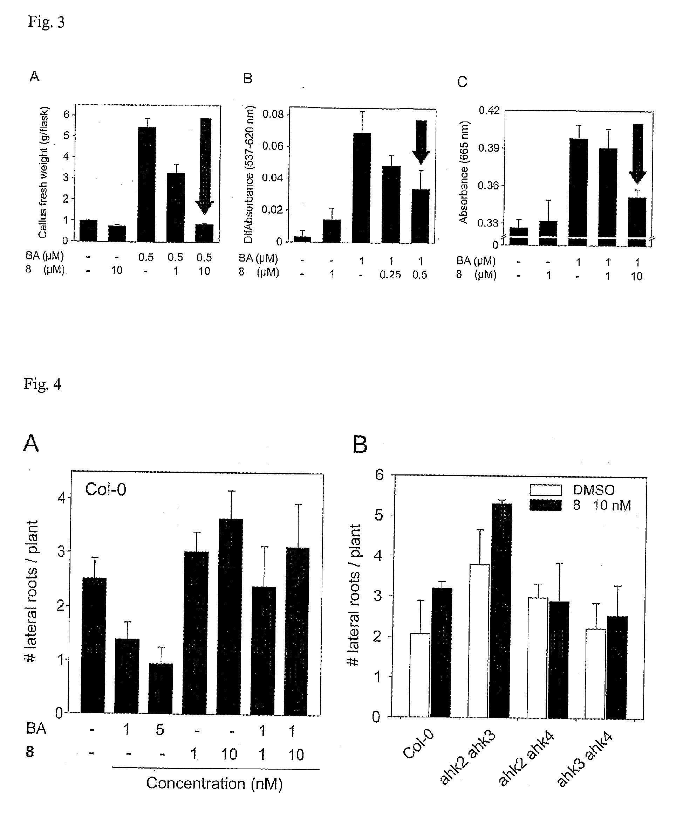

Figure 3 ilustrates antagonistic effect of compound 8 in standard cytokinin bioassays (A) Effect on cell proliferation of cytokinin-dependent *Arabidopsis* callus. (B) Effect on cytokinin stimulated betacyanin formation in the dark in *Amaranthus* hypocotyls. (C) Effect on cytokinin-induced retention of chlorophyll in excised wheat leaves.
Figure 4 shows *in vivo* effect of compound 8 on lateral root formation. (A) Number of lateral roots formed by wild-type Arabidopsis seedlings (11 DAG) - from left: DMSO control, formation in the presence of cytokinin BA, or compound 8, and antagonism when both compounds were added. (B) Increase in number of lateral roots found with WT and double-receptor mutant seedlings grown on medium containing compound 8.

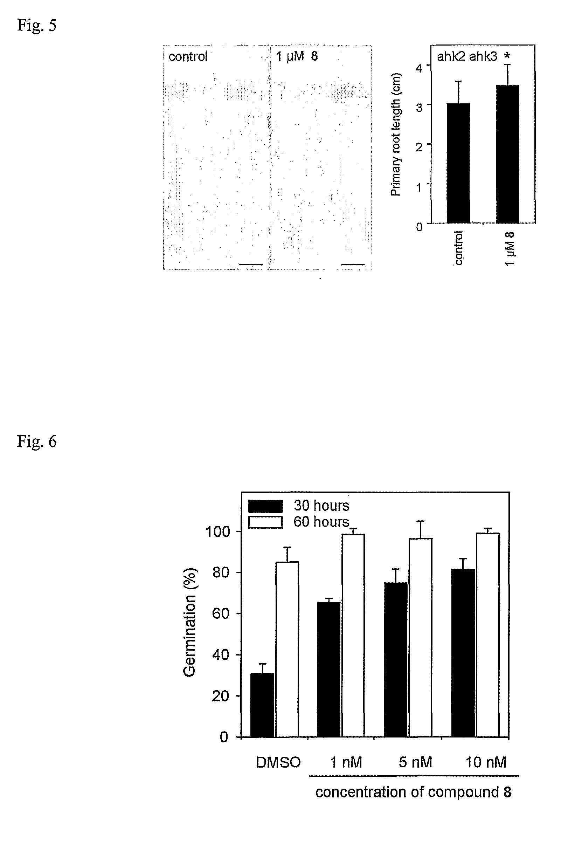

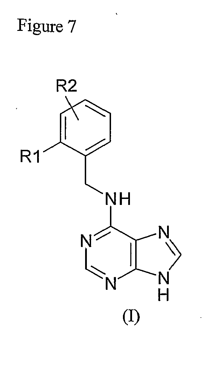

Figure 5 Effect of compound 8 on primary root length of *Arabidopsis* seedlings. Increase in the length of the primary roots observed in *ahk2 ahk3* double mutant seedlings (6 DAG) grown on medium containing 1 µM compound 8.
Figure 6 shows *in vivo* effect of compound 8 on germination. Early germination of wild-type Arabidopsis seeds cultivated on MS medium containing indicated concentrations of compound 8 after replacement from dark to white light (16 h light/8 hours dark).

### Examples of carrying out the Invention

The starting material for the compounds of the formula **I** is 6-chloropurine or 6-bromopurine. Starting substituted benzylamines, not commercially available (others obtained *via* Sigma Aldrich or Fluorochem), were prepared from the corresponding aldehydes in the presence of suitable metal catalyst. Those, which have one or more methyl groups, may also be prepared from corresponding methylbenzaldehydes. Hydroxyderivatives are prepared by demethylation of corresponding methoxyderivatives using 48% HBr in N₂ atmosphere.

Elemental analyses (C, H and N) were performed on an EA1108 CHN analyser (Fissons Instruments). The melting points were determined on a BÜCHI Melting Point B-540 apparatus. Analytical thin layer chromatography (TLC) was carried out using silica gel 60 WF₂₅₄ plates (Merck), solvent CHCl₃:MEOH:conc. NH₄OH (8:2:0.2, v/v/v). ES+ mass spectra were recorded using direct probe on Waters ZMD 2000 mass spectrometer. The mass monitoring interval was 10 - 1500 amu. The spectra were collected using 3.0 second cyclical scans and applying sample cone voltage 25 V at source block temperature 150 °C, desolvation temperature 80 °C and desolvation gas flow rate 200 l/hour. The mass spectrometer was directly coupled to a MassLynx data system. NMR spectra were measured in a Bruker Avance AV 300 spectrometer operating at a temperature of 300 K and a frequency of 300.3 MHz (¹H) and 75.48 MHz (¹³C), respectively. Samples were prepared by dissolving the compounds in DMSO-d₆. Tetramethylsilane (TMS) was used as the internal standard.

### EXAMPLE 1

### Preparation of 6-(2-hydroxy-3-methylbenzylamino)purine

3 mmol 6-chloropurine were disolved in 15 ml butanol and 4 mmol 2-hydroxy-3-methylbenzylamine and 5 mmol triethylamine were added. The solution was kept at 90 °C for 4 hours. After cooling to room temperature the precipitate was filtered off and recrystallised from ethanol. M.p. 276-277 °C. TLC: chloroform-methanol-ammonia (90:9:1): no impurities and starting material; HPLC purity: 98+ %. Yield 92 %.

**Table 1**

| Compounds Prepared by the method of Example 1 | | | | |
|---|---|---|---|---|
| PREPARED COMPOUNDS | | CHN ANALYSES | MS ANALYSES -ZMD | |
| | | [%] | [M-H]⁻ a) | [M+H]⁺ b) |
| **1** | 6-(2-amino-3-methylbenzylamino)purine | C=60.8; H=5.7; N=32.7 | 253 | 255 |
| **2** | 6-(2-amino-4-methylbenzylamino)purine | C=61.2; H=5.6; N=32.9 | 253 | 255 |
| **3** | 6-(2-amino-5-methylbenzylamino)purine | C=61.2; H=5.6; N=32.9 | 253 | 255 |
| **4** | 6-(2-amino-3-ethylbenzylamino)purine | C=62.4; H=6.0; N=31.1 | 267 | 269 |
| **5** | 6-(2-amino-5-ethylbenzylamino)purine | C=62.5; H=6.0; N=31.2 | 267 | 269 |
| **6** | 6-(2-amino-3-isopropylbenzylamino)purine | C=63.8; H=6.1; N=29.4 | 281 | 283 |
| **7** | 6-(2-amino-5-isopropylbenzylamino)purine | C=63.6; H=6.3; N=29.8 | 281 | 283 |
| **8** | 6-(2-hydroxy-3-methylbenzylamino)purine | C=60.9; H=5.4; N=27.3 | 254 | 256 |
| **9** | 6-(2-hydroxy-4-methylbenzylamino)purine | C=60.5; H=5.3; N=27.2 | 254 | 256 |
| **10** | 6-(2-hydroxy-5-methylbenzylamino)purine | C=60.8; H=5.3; N=27.4 | 254 | 256 |
| **11** | 6-(2-hydroxy-6-methy(benzylamino)purine | C=60.8; H=5.3; N=27.2 | 254 | 256 |
| **12** | 6-(2-hydroxy-3-ethylbenzylamino)purine | C=62.4; H=5.5; N=25.9 | 268 | 270 |
| **13** | 6-(2-hydroxy-5-ethylbenzylamino)purine | C=62.3; H=5.5; N=25.8 | 268 | 270 |
| **14** | 6-(2-hydroxy-3-isopropylbenzylamino)purine | C=63.6; H=6.2; N=24.5 | 282 | 284 |
| **15** | 6-(2-nitro-3-methylbenzylamino)purine | C=54.7; H=4.3; N=29.3 | 283 | 285 |
| **16** | 6-(2-nitro-5-methylbenzylamino)purine | C=54.8; H=4.3; N=29.3 | 283 | 285 |
| **17** | 6-(2-nitro-3-ethylbenzylamino)purine | C=56.3; H=4.7; N=28.2 | 297 | 299 |
| **18** | 6-(2-thio-3-methylbenzylamino)purine | C=57.4; H=4.7; N=25.6 | 270 | 272 |
| **19** | 6-(2-thio-5-methylbenzylamino)purine | C=57.3; H=4.7; N=25.7 | 270 | 272 |
| **20** | 6-(2-thio-3-ethylbenzylamino)purine | C=58.3; H=5.2; N=24.6 | 284 | 286 |
| **21** | 6-(2-amino-3,5-dimethylbenzylamino)purine | C=62.5; H=5.9; N=30.9 | 267 | 269 |
| **22** | 6-(2-amino-3,6-dimethylbenzylamino)purine | C=62.3; H=5.8; N=30.7 | 267 | 269 |
| **23** | 6-(2-hydroxy-3,5-dimethylbenzylamino)purine | C=62.2; H=5.5; N=25.8 | 268 | 270 |
| **24** | 6-(2-hydroxy-3,4-dimethylbenzylamino)purine | C=62.3; H=5.5; N=25.9 | 268 | 270 |
| **25** | 6-(2-hydroxy-3,6-dimethylbenzylamino)purine | C=62.2; H=5.6; N=25.8 | 268 | 270 |
| **26** | 6-(2-hydroxy-3,4,5-trimethylbenzylamino)purine | C=63.2; H=5.9; N=24.5 | 282 | 284 |
| **27** | 6-(2-hydroxy-3,5-diethylbenzylamino)purine | C=64.0; H=6.6; N=23.2 | 296 | 298 |

### EXAMPLE 2

### Agonistic activity on cytokinin receptors

*Escherichia coli* strains KMI001 harbouring the plasmid pIN-III-AHK4 or pSTV28-AHK3 were grown overnight at 25 °C in M9 media enriched with 0.1% casamino acids to OD₆₀₀∼1. The preculture was diluted 1:600 in 1 ml M9 medium containing 0.1% casamino acids and 1 µl stock solution of either the tested compound (10⁻⁷ M - 5 x 10⁻⁵ M) or solvent control (DMSO, ethanol, methanol) were added. The cultures were further grown at 25 °C in microtiter plate, 200 µl per well. Incubation times of 17 h and 28 h were found to be optimal for CRE1/AHK4 and AHK3, respectively. The cultures were centrifuged and 50 µl aliquots of the supernatant were transferred to microtiter plate containing 2 µl 50 mM 4-methyl umbelliferyl galactoside which was subsequently incubated for 1 h at 37 °C. The reaction was stopped by adding 100 µl 0.2 M Na₂CO₃. Fluorescence was measured using a Fluoroscan Ascent (Labsystems, Finland) at the excitation and emission wavelengths of 365 and 460 nm, respectively. The OD₆₀₀ of remaining culture was determined and β-galactosidase activity was calculated as nmol 4-methylumbelliferone x OD₆₀₀⁻¹ x h⁻¹.

The EC₅₀ value, the compound concentration activating the receptor to 50 %, was calculated from the obtained dose response curves. The compound functioning as cytokinin antagonists should not activate cytokinin signalling pathway. The values shown in Table 2 are means of three replicates and the entire test was repeated at least twice. Newly prepared substituted 6-(alkylbenzylamino)purine derivatives embodied much lower affinity to *A. thaliana* cytokinin receptors than the control cytokinin *trans*-zeatin.

**Table 2**

| The effect of novel compounds on activation of cytokinin receptors of *Arabidopsis thaliana* CRE1/AHK4 and AHK3. | | | |
|---|---|---|---|
| No. | Tested compound | EC50 (µmol.L⁻¹) | |
| | | CRE1/AHK4 | AHK3 |
| | *trans*-zeatin | 0.9 | 2.1 |
| | 6-(benzylamino)purine | 19.7 | 18.2 |
| **1** | 6-(2-amino-3-methylbenzylamino)purine | n.i. | n.i |
| **2** | 6-(2-amino-4-methylbenzylamino)purine | n.i. | n.i |
| **3** | 6-(2-amino-5-methylbenzylamino)purine | n.i. | n.i |
| **4** | 6-(2-amino-3-ethylbenzylamino)purine | n.i. | n.i |
| **5** | 6-(2-amino-5-ethylbenzylamino)purine | n.i. | n.i |
| **8** | 6-(2-hydroxy-3-methylbenzylamino)purine | n.i. | >100 |
| **9** | 6-(2-hydroxy-4-methylbenzylamino)purine | n.i. | n.i |
| **10** | 6-(2-hydroxy-5-methylbenzylamino)purine | n.i. | >100 |
| **11** | 6-(2-hydroxy-6-methylbenzylamino)purine | n.i. | n.i |
| **12** | 6-(2-hydroxy-3-ethylbenzylamino)purine | n.i. | n.i |
| **13** | 6-(2-hydroxy-5-ethylbenzylamino)purine | n.i. | n.i |
| **15** | 6-(2-nitro-3-methylbenzylamino)purine | n.i. | >100 |
| **16** | 6-(2-nitro-5-methylbenzylamino)purine | n.i. | n.i |
| **17** | 6-(2-nitro-3-ethylbenzylamino)purine | n.i. | n.i |
| **19** | 6-(2-thio-5-methylbenzylamino)purine | n.i. | n.i |
| **21** | 6-(2-amino-3,5-dimethylbenzylamino)purine | n.i. | n.i |
| **23** | 6-(2-hydroxy-3,5-dimethylbenzylamino)purine | n.i. | n.i |
| **24** | 6-(2-hydroxy-3,4-dimethylbenzylamino)purine | n.i. | n.i |

| | | | |
|---|---|---|---|
| **n.i.** means without interaction | | | |

### EXAMPLE 3

### Inhibition of binding of natural ligand to cytokinin receptor by 6-(2-hydroxy-3-methylbenzylamino)purine (compound 8)

For the binding assay membranes isolated from *E. coli* expressing cytokinin receptors CRE1/AHK4 and AHK3 were used (see example 2). Isolation of *E. coli* membranes and binding assays were carried out as previously described by Romanov et al. (Romanov et al. Analytical Biochemistry 347:129-134, 2005). In the assay the influence of increasing concentration of competitor (compound **8**) on binding of radiolabeled natural ligand *trans*-zeatin (³HtZ) was tested. Non-labeled *trans*-zeatin (tZ) was used as positive and adenine as negative controls. Compound **8** was able to decrease the binding of 3HtZ to 50% in 3 µM concentration (Fig. 1), whereas even 1000-fold higher concentration of adenine was not effective at all.

### EXAMPLE 4

### Decrease of induction of cytokinin reporter ARR5 expression after application of cytokinin antagonist 6-(2-hydroxy-3-methylbenzylamino)purine (compound 8)

Cytokinins induce in *Arabidopsis* transcription of gene *ARR5,* a member of A-type response regulator family classified as cytokinin primary response genes. We cultivated transgenic *Arabidopsis* plants harbouring *P_{ARR5}::GUS* reporter (D'Agostino et al. Plant Physiol. 124: 1706-1717, 2000) in MS medium containing 2.5 µM 6-benzylaminopurine (BA) in the presence, or absence of 5 µM compound **8;** DMSO (0.1%) was tested as solvent control. Seeds were surface-sterilized in 70% ethanol and then placed into wells of 6-well microtiter plate (TPP, Switzerland) containing 3 mL of MS medium in each well. After sowing plates were pretreated in dark at 4°C to synchronize the germination of the seeds. Then the plates were transferred to growth chamber (22 °C, 16h light/8h dark) and after 2-3 days after germination the tested compounds were applied directly into the media. The plants were incubated with the tested compounds for 4 h and 17 h in the case of the qualitative or quantitative estimation, respectively. Qualitative estimation - GUS staining was done according to Jefferson et al. (Jefferson et al. EMBO J. 6: 3901-3907, 1987). Endogenous pigments of the plants were first destained with 90% acetone (1 h, 4 °C) and then the GUS staining was performed (40 min, 37 °C) with X-Glc (5-bromo-4-chloro-3-indolyl-β-D-glucuronid, sodium salt) as substrate. The reaction was stopped by transferring the plants into 70% ethanol. As shown in Fig. 2A compound **8** decreased expression of ARR5:GUS induced by cytokinin in the root and completely inhibited BA-induced reporter gene expression in the shoot. Quantitative estimation of the level of *ARR5:GUS* gene induction was done according to method published by Romanov et al. (Romanov et al. FEBS Letters 515: 39-43, 2002). After extraction of proteins the GUS activity was determined using incubation with fluorogenic substrate MUG (4-methylumbelliferyl glucuronid; 1 h, 37 °C) and then fluorescence 365 nm and 460 nm (excitation and emission wavelengths) was recorded, as described in detail by Spichal et al. (Spíchal et al. Plant and Cell Physiology 45: 1299-1305, 2004). Quantitative assay confirmed compound **8** dose-dependent decrease of ARR5:GUS expression (Fig. 2B). This confirms that perception of cytokinin by the specific receptor as the event that is localized "up-stream" of the response to cytokinin was inhibited.

### EXAMPLE 5

### Testing of the effect of novel compounds on plant cell division

Stimulatory effect of newly prepared derivatives was tested using cytokinin-dependent tobacco callus. The cytokinin-dependent tobacco callus *Nicotiana tabacum* L. cv. Wisconsin 38 was maintained at 25 °C in darkness on modified MS medium, containing per 1 liter: 4 µmol of nicotinic acid, 2.4 µmol of pyridoxine hydrochloride, 1.2 µmol of thiamine, 26.6 µmol of glycine, 1.37 µmol of glutamine, 1.8 µmol of myo-inositol, 30 g of sucrose, 8 g of agar, 5.37 µmol of NAA and 0.5 µmol of the compound tested. Subcultivation was carried out every three weeks. Fourteen days before the bioassay, the callus tissue was transferred to the media without 6-benzylaminopurine. The biological activity was determined from the increase of the fresh callus weight after four weeks of cultivation. Five replicates were prepared for each concentration of the compound tested and the entire test was repeated twice. Kinetin, which is known to be highly active cytokinin, was used in each experiment as a control. The compounds to be tested were dissolved in dimethylsulfoxide (DMSO) and the solution brought up to 10⁻³ M with distilled water. This stock solution was further diluted with the respective media used for the biotest to a concentration ranging from 10⁻⁸ M to 10⁻⁴ M. The final concentration of DMSO in the medium did not exceed 0.2 % and therefore did not affect the biological activity in the assay system used.

From the obtained data, the concentration with the highest activity was selected for each compound tested. Relative activity of the compound at this concentration was calculated (Table 3). The activity obtained for 10⁻⁶ M of the control substance 6-benzylaminopurine was postulated as 100 % biological activity.

Compounds functioning as cytokinin antagonist should not exhibit stimulatory effect on cell divion of plant cells. The results in Table 3 show that the newly prepared substituted 6-(alkylbenzylamino)purine derivatives of the general formula **I** showed strong decrease or complete loss of the cytokinin activity in the callus bioassay in comparison to the classical cytokinin - 10⁻⁶ M concentration of the control substance 6-benzylaminopurine was postulated as 100 %.

**Table 3**

| The effect of novel compounds on the growth of cytokinin-dependent tobacco callus *Nicotiana tabacum* L. cv. Wisconsins 38 | | | |
|---|---|---|---|
| No | Tested compound | concentration with highest activity (mol.l⁻¹) | activity (%) [10⁻⁶mol.l⁻¹ BA = 100%] |
| | 6-(benzylamino)purine | 10⁻⁶ | 100 |
| **1** | 6-(2-amino-3-methylbenzylamino)purine | | n.a. |
| **2** | 6-(2-amino-4-methylbenzylamino)purine | | n.a. |
| **3** | 6-(2-amino-5-methylbenzylamino)purine | | n.a. |
| **4** | 6-(2-amino-3-ethylbenzylamino)purine | | n.a. |
| **5** | 6-(2-amino-5-ethylbenzylamino)purine | | n.a. |
| **8** | 6-(2-hydroxy-3-methylbenzylamino)purine | 10⁻⁴ | 2 (±2) |
| **9** | 6-(2-hydroxy-4-methylbenzylamino)purine | | n.a. |
| **10** | 6-(2-hydroxy-5-methylbenzylamino)purine | 10⁻⁴ | 35 (±8) |
| **11** | 6-(2-hydroxy-6-methylbenzylamino)purine | 10⁻⁴ | 23 (±5) |
| **12** | 6-(2-hydroxy-3-ethylbenzylamino)purine | | n.a. |
| **13** | 6-(2-hydroxy-5-ethylbenzylamino)purine | | n.a. |
| **15** | 6-(2-nitro-3-methylbenzylamino)purine | | n.a. |
| **16** | 6-(2-nitro-5-methylbenzylamino)purine | | n.a. |
| **17** | 6-(2-nitro-3-ethylbenzylamino)purine | | n.a. |
| **19** | 6-(2-thio-5-methylbenzylamino)purine | 10⁻⁴ | 1.3 (±1) |
| **21** | 6-(2-amino-3,5-dimethylbenzylamino)purine | | n.a. |
| **23** | 6-(2-hydroxy-3,5-dimethylbenzylamino)purine | | n.a. |
| **24** | 6-(2-hydroxy-3,4-dimethylbenzylamino)purine | | n.a. |

| | | | |
|---|---|---|---|
| **n.a.** means inactive | | | |

### EXAMPLE 6

### Testing of novel compounds in Amaranthus bioassay

The standard *Amaranthus* bioassay with several modifications was used to study cytokinin activity. The seeds of *Amaranthus caudatus* var. *atropurpurea* were surface-sterilised in 10% (w/v) N-chlorobenzenesulfonamide for 10 min and washed 5 times with deionized water. They were placed in 14 cm Petri dishes containing paper tissues saturated with deionized water. After 72 h of cultivation at 25 °C in darkness, the roots of the seedlings were cut off. The explants, consisting of two cotyledons and hypocotyl, were placed in 5 cm Petri dishes onto two layers of filter paper soaked with 1 ml of the incubation medium containing 10 µmol of NA₂HPO₄- KH₂PO₄, pH 6.8, 5 µmol of tyrosine and the cytokinin to be tested. There were 20 explants per dish. The procedure was carried out under a green safe light in a darkroom. After 48 h of incubation at 25 °C in darkness, betacyanin was extracted by freezing the explants in 4 ml 3.33 µM acetic acid. The concentration of betacyanin was determined from the absorbances at 537 nm and 620 nm as follows: ΔA = A₅₃₇ₙₘ - A₆₂₀ₙₘ. The values ΔA were plotted against the concentration tested, are means of five replicates and the entire test was repeated twice. 6-Benzylaminopurine, which is known to be highly active cytokinin, was used in each experiment as a control. The compounds to be tested were dissolved in dimethylsulfoxide (DMSO) and the solution brought up to 10³ M with distilled water. This stock solution was further diluted with the respective media used for the biotest to a concentration ranging from 10⁻⁸ M to 10⁻⁴ M. The final concentration of DMSO did not exceed 0.2 % and therefore did not affect the biological activity in the assay system used. The activity obtained for 10⁻⁴ M 6-benzylaminopurine was postulated as 100 %.

The compound functioning as cytokinin antagonist should not exhibit stimulatory effect on betacyanin production in amaranthus. Similarly to callus bioassay the newly prepared substituted 6-(alkylbenzylamino)purine derivatives of the general formula I showed strong decrease or complete loss of the cytokinin activity in comparison to the classical cytokinin 6-benzylaminopurine (BA).

### EXAMPLE 7

### Antisenescencent activity of novel compounds tested in senescent bioassay on wheat leaf segments

Seeds of winter wheat, *Triticum aestivum* cv. Hereward, were washed under running water for 24 hours and then sown on vermiculite soaked with Knop's solution. They were placed in the growth chamber at 25°C with a 16/8 h light period at 50 µmol.m⁻².s⁻¹. After 7 days, the first leaf was fully developed and the second leaf had started to grow. A tip section of the first leaf, approximately 35 mm long, was removed from 5 seedlings and trimmed slightly to a combined weight of 100 mg. The basal ends of the five leaf tips were placed in the wells of a microtiter polystyrene plate containing 150 µL of the tested derivative solution each. The entire plate was inserted into a plastic box lined with paper tissues soaked in distilled water to prevent leaf sections from drying out. After 96 h incubation in the dark at 25°C, the leaves were removed and chlorophyll extracted by heating at 80°C for 10 min in 5 mL of 80% ethanol (v/v). The sample volume was then restored to 5 mL by the addition of 80% ethanol (v/v). The absorbance of the extract was recorded at 665 nm. In addition, chlorophyll extracts from fresh leaves and leaf tips incubated in deionised water were measured. The results are means of five replicates and the entire test was repeated twice. 6-Benzylaminopurine, which is known to be highly active cytokinin, was used in each experiment as a control. The compounds to be tested were dissolved in dimethylsulfoxide (DMSO) and the solution brought up to 10⁻³ M with distilled water. This stock solution was further diluted with the respective media used for the biotest to a concentration ranging from 10⁻⁸ M to 10⁻⁴ M. The final concentration of DMSO did not exceed 0.2 % and therefore did not affect the biological activity in the assay system used. The activity obtained for 10⁻⁴ M 6-benzylaminopurine was postulated as 100 %.

The compound functioning as cytokinin antagonist should not exhibit positive effect on delaying of senescence wheat leaf segments. Similarly to callus bioassay the newly prepared substituted 6-(alkylbenzylamino)purine derivatives of the general formula **I** showed strong decrease or complete loss of the cytokinin activity in comparison to the classical cytokinin 6-benzylaminopurine (BA).

### EXAMPLE 8

### Antagonistic effect of 6 -(2-hydroxy-3-methylbenzylamino)purine (compound 8) in cytokinin-mediated processes

The above described classical cytokinin bioassays (callus, amaranthus, senescence) were further used for confirmation of *in vitro* antagonistic effect of compound **8** in cytokinin-mediated processes. In the assays 0.5-1 µM BA was used to induce the cytokinin-dependent action. Compound **8** was applied either alone in 1 µM concentration to test the intrinsic cytokinin activity, or in wider range (10 nM - 10 µM) in combination with BA for testing the antagonistic effect. As illustrated in Fig. 3, in all the biotests used the compound significantly decreased biological effect of active cytokinin 6-benzylaminopurine (BA) that was applied in its optimal concentration. The strength of antagonistic effect was more pronounced with increasing concentration of compound **8.**

### EXAMPLE 9

### In vivo effect of 6-(2-hydroxy-3-methylbenzylamino)purine (compound 8) on enhancement of root system of model Arabidopsis plants

Cytokinins are known as negative regulators of root growth and development. We grew *A. thaliana* wild-type and double receptor mutant seedlings with decreased perception of cytokinins on MS medium containing either 1 and 5 nM cytokinin BA to confirm the cytokinin inhibitory effect on initiation of lateral roots, or 1 and 10 nM compound **8,** or combination of these substances in concentrations indicated in Fig. 4A. As shown in Fig. 4A, cytokinin inhibitited formation of lateral roots. Clear antagonistic effect was observed when root branching was inspected with 14 DAG old plants grown on medium supplemented with compound **8.** Interestingly, the compound **8** showed positive effect on root branching even when applied alone in submicromolar concentrations, indicating that also action of endogenous cytokinins was suppressed in the roots of model plants. In receptor mutants, which has only one of three receptors functional, the effect was best seen with *ahk2ahk3* mutants. this confirms at *in vivo* level that compound **8** primarly blocks the binding to the receptor AHK4.

Compound **8** alone did not affect the elongation of the primary root of wild-type *Arabidopsis* seedlings; neither positive but importantly nor negative effects were observed even at 1 µM. In contrast, 1 nM BA almost completely inhibited primary root growth (data not shown). However, when 1 µM compound **8** was applied to the growth media of *ahk2 ahk3* double mutants, significant increase (about 15 %) in the length of the primary roots was observed compared to untreated control (Fig. 5). No significant changes in elongation of the primary root were found with other two receptor double mutants (data not shown). This result is consistent with a predominant activity of compound **8** on CRE1/AHK4 and indicates that this receptor has a regulatory function in the control of primary root elongation.

### EXAMPLE 10

### In vivo effect of 6-(2-hydroxy-3-methylbenzylamino)purine (compound 8) on germination of seeds of model Arabidopsis plants

During the root assaying positive effect of the compound **8** on shortening of the time of germination was observed with wild-type *Arabidopsis* seeds. Subsequent testing confirmed positive effect of the compound **8** on germination. After sowing and pretreatment in dark at 4°C, seeds were transferred to light and ambient temperature. After 30 hours more than 60 % of seeds sown on medium containing 1 nM compound **8** were germinating, which was twice as much as in the case of control sown on compound **8** free medium. The number of germinating seeds was further enhanced with higher concentration of compound **8** and 80 % of seeds were germinated when 10 nM compound **8** was present in the medium (Fig. 6).

This developmental effect as well as the effect described in the example 9 is similar to phenotype observed with receptor mutant plants with decreased cytokinin perception (Riefler et al., Plant Cell 18:40-45, 2006).

### EXAMPLE 11

### In vitro cytotoxic activity of novel compounds

Absence of toxic effects against mammalian (especially human) cell lines in a wide concentration range is one of the requirements on compounds intended for use in agriculture. Because toxic compounds negatively influence metabolic processes in cells, many standard cytotoxicity assays are based on measurement of metabolisation rate of various artificial substrates. Resulting product is then quantified e.g. by means of spectrometry. The assays can be easily modified for use in 96-well plates. For evaluation of cytotoxic effect of the novel substituted 6-(alkylbenzylamino)purine derivatives of this invention, a microtiter assay based on quantification of metabolisation of Calcein AM was used. The assay is widely used in drug screening programs and in chemosensitivity testing. In live cells, Calcein AM is enzymatically hydrolysed and accumulation of resulting calcein is manifested by green fluorescence.

Following human cell lines were used for rutine screening of the compounds: human diploid fibroblasts BJ, erytroid leukemia cell line K-562, breast carcinoma cell line MCF-7, osteosarcoma cell line HOS and melanoma cell line G-361.

The cells were maintained in Nunc/Corning 80 cm² plastic tissue culture flasks and cultured in cell culture medium (DMEM with 5 g/l glucose, 2mM glutamine, 100 U/ml penicillin, 100 µg/ml streptomycin, 10% fetal calf serum and sodium bicarbonate).

The cell suspensions were prepared and diluted according to the particular cell type and the expected target cell density (10⁴ cells per well based on cell growth characteristics) and pippetted (80µl) into 96-well plates. Inoculates were allowed a pre-incubation period of 24 hours at 37 °C, 100 % humidity and 5% CO₂ for stabilisation. Tested compounds were added in total volume of 20 µl of water at time zero. Usually, test compound was evaluated at six 3-fold dilutions. In routine testing, the highest concentration tested was 100 µM, the eventual changes depended on physico-chemical characteristics of the respective compound. All drug concentrations were tested in triplicates. Incubations of cells with the test compounds lasted for 72 hours at 37 °C, in 5% CO₂ atmosphere and 100% humidity. At the end of incubation period Calcein AM (Molecular Probes) in PBS was added into final concentration of 1 µg / ml. After another 1 hour of incubation fluorescence (FD) was measured with the Labsystem FIA Reader Fluoroscan Ascent (UK). Growth inhibition (GI) was estimated using the following equitation: GI = (mean FD_{drug exposed wells} - mean FD_{blank}) / (mean FD_{control wells}- mean FD_{blank}) x 100%. The GI₅₀ value, the drug concentration causing 50% reduction of Calcein AM conversion, was calculated from the obtained dose response curves. The results are shown in Table 4.

**Table 4**

| Cytotoxicity of the novel compounds against human cell lines | | | | | | |
|---|---|---|---|---|---|---|
| Compound | Cell line / GI₅₀ (µmol/L) | | | | | Maximum concentration tested (µmol/L) |
| | BJ | K-562 | MCF7 | HOS | G-361 | |
| kinetin | >100 | >100 | >100 | >100 | >100 | 100 |
| isopentenyladenine | >100 | >100 | >100 | >100 | >100 | 100 |
| benzyladenine | >100 | >100 | >100 | >100 | >100 | 100 |
| *trans*-zeatin | >100 | >100 | >100 | >100 | >100 | 100 |
| *meta*-topolin | >100 | >100 | >100 | >100 | >100 | 100 |
| *ortho*-topolin | >100 | >100 | >100 | >100 | >100 | 100 |
| Adenine | >100 | >100 | >100 | >100 | >100 | 100 |
| **8** | >50 | >50 | >50 | >50 | >50 | 50 |
| **10** | >100 | >100 | >100 | >100 | >100 | 100 |

The novel 6-(alkylbenzylamino)purine derivatives show no toxicity to normal and tumour cells in the concentration range tested and thus are suitable for agricultural applications.

### Example 12

### Evaluation of the effect on viability of human diploid fibroblasts by MTT

MTT is a standard colorimetric assay for measurement of proliferation and survival of the cells. Yellow MTT is reduced into violet formazan in metabolically active cells. The amount of formazan is measured by spectrometry. Human diploid fibroblasts SNF25 (passage 19) were seeded in 96-well plate (5000 cells per well). After 6 hours, the cultivation medium (DMEM containing 5 g/l glucose, 2 mM glutamin, 100 U/ml penicillin, 100 µg/ml streptomycin and 10% fetal calf serum) was removed and fresh medium containing a test compound in concentration range of 0-100 µM was added. The concentration was adjusted in cases of compounds with limited solubility. Each concentration was tested in 5 replicates. MTT was added to the cells after 72 hours into final concentration of 0.5 mg/ml. Incubation time was 3 hours. Resulting MTT was disolved in DMSO and absorbance was measured. The cytotoxic effect was estimated as IC = (A_{wells} with_{compounds}/A_{control} wells) x 100 %. Parameter IC₅₀ corresponding to the compound concentration causing 50 % reduction in mitochondrial activity was calculated from dose response curves. The results are summarised in the Table 5.

**Table 5**

| Cytotoxicity of the novel compounds against human diploid fibroblasts | | |
|---|---|---|
| Compound | IC₅₀ (µmol/L) | Maximum concentration tested (µmol/L) |
| kinetin | >100 | 100 |
| isopentenyladenine | >100 | 100 |
| benzyladenine | >100 | 100 |
| *trans*-zeatin | >100 | 100 |
| *meta*-topolin | >100 | 100 |
| *ortho*-topolin | >100 | 100 |
| adenine | >100 | 100 |
| **8** | >50 | 50 |
| **10** | >100 | 100 |

The compounds tested are not toxic for human diploid fibroblasts in concentrations supposed to be used in agricultural applications.

### EXAMPLE 13

### Formulations

The growth regulatory formulations usually contain from 0.1 to 99 % (w/w), especially from 0.1 to 95 % (w/w), of active ingredient mixture comprising a compound of formula I, from 1 to 99.9% (w/w), of a solid or liquid formulation adjuvant, and from 0.1 to 25 % (w/w), especially from 0.1 to 25 % (w/w), of a surfactant. Whereas commercial products are usually formulated as concentrates, the end user will normally employ dilute formulations. The compositions may also comprise further ingredients, such as stabilisers, e.g. vegetable oils or epoxidised vegetable oils (epoxidised coconut 0;1, rapeseed oil or soybean oil), antifoams, e.g. silicone oil, preservatives, viscosity regulators, binders, tackifiers, and also fertilisers or other active ingredients. Preferred formulations have especially the following compositions: (% = percent by weight)

| A1. Emulsifiable concentrates | a) | b) | c) | d) |
|---|---|---|---|---|
| active ingredient | 5% | 10% | 25% | 50% |
| calcium dodecylbenzenesulfonate | 6% | 8% | 6% | 8% |
| castor oil polyglycol ether (36 mol of ethylene oxide) | 4% | - | 4% | 4% |
| octylphenol polyglycol ether (7-8 mol of ethylene oxide) | 2% | - | 2% | - |
| cyclohexanone | | - | 10% | 20 % |
| arom. hydrocarbon mixture | 83% | 82% | 53 % | 18% |

C₉-C₁₂
Emulsions of any desired final concentration can be obtained from such concentrates by dilution with water.

| A2. Solutions | a) | b) | c) | d) |
|---|---|---|---|---|
| active ingredient | 5% | 10% | 50% | 90% |
| 1-methoxy-3-(3-methoxy-propoxy)-propane | - | 20 % | 20% | - |
| polyethylene glycol MW 400 | 20% | 10% | - | - |
| N-methyl-2-pyrrolidone | - | - | 30% | 10% |
| arom. hydrocarbon mixture | 75% | 60 % | - | - |

C₉-C₁₂
The solutions are suitable for use in the form of microdrops.

| A3. Wettable powders | a) | b) | c) | d) |
|---|---|---|---|---|
| active ingredient | 5% | 25% | 50% | 80 % |
| sodium lignosulfonate | 4% | | 3% | - |
| sodium lauryl sulfate | 2% | 3% | | 4 % |
| sodium diisobutylnaphthalene-sulfonate | - | 6% | 5% | 6 % |
| octylphenol polyglycol ether (7-8 mol of ethylene oxide) | 1% | 2% | - | - |
| highly dispersed silicic acid | 1% | 3% | 5% | 10 % |
| kaolin | 87% | 61% | 37% | - |

The active ingredient is mixed thoroughly with the adjuvants and the mixture is thoroughly ground in a suitable mill, affording wettable powders which can be diluted with water to give suspensions of any desired concentration.

| A4. Coated granules | a) | b) | c) |
|---|---|---|---|
| active ingredient | 0.1 % | 5% | 15% |
| highly dispersed silicic acid | 0.9 % | 2 % | 2 % |
| inorganic carrier | 99.0 % | 93 % | 83 % |

(0.1 -1 mm)
e.g. CaCO₃ or SiO₂
The active ingredient is dissolved in methylene chloride and applied to the carrier by spraying, and the solvent is then evaporated off in vacuo.

| A5. Coated granules | a) | b) | c) |
|---|---|---|---|
| active ingredient | 0.1 % | 5 % | 15 % |
| polyethylene glycol MW 200 | 1.0 % | 2 % | 3 % |
| highly dispersed silicic acid | 0.9 % | 1 % | 2 % |
| inorganic carrier | 98.0 % | 92 % | 80 % |

(AE 0.1 -1 mm)
e.g. CaCO₃ or SiO₂
The finely ground active ingredient is uniformly applied, in a mixer, to the carrier moistened with polyethylene glycol. Non-dusty coated granules are obtained in this manner.

| A6. Extruder granules | a) | b) | c) | d) |
|---|---|---|---|---|
| active ingredient | 0.1 % | 3 % | 5 % | 15 % |
| sodium lignosulfonate | 1.5 % | 2 % | 3 % | 4 % |
| carboxymethylcellulose | 1.4 % | 2 % | 2 % | 2 % |
| kaolin | 97 % | 93 % | 90 % | 79 % |

The active ingredient is mixed and ground with the adjuvants, and the mixture is moistened with water. The mixture is extruded and then dried in a stream of air.

| A7. Dusts | a) | b) | c) |
|---|---|---|---|
| active ingredient | 0.1 % | 1 % | 5 % |
| talc | 39.9 % | 49 % | 35 % |
| kaolin | 60 % | 50 % | 60 % |

Ready-to-use dusts are obtained by mixing the active ingredient with the carriers and grinding the mixture in a suitable mill.

| A8. Suspension concentrates | a) | b) | c) | d) |
|---|---|---|---|---|
| active ingredient | 3 % | 10 % | 25 % | 50 % |
| ethylene glycol | 5 % | 5 % | 5 % | 5 % |
| nonylphenol polyglycol ether (15 mol of ethylene oxide) | 1 % | 2 % | - | - |
| sodium lignosulfonate | 3 % | 3 % | 4 % | 5 % |
| carboxymethylcellulose | 1 % | 1 % | 1 % | 1 % |
| 37 % aqueous formaldehyde solution | 0.2 % | 0.2 % | 0.2 % | 0.2 % |
| silicone oil emulsion | 0.8 % | 0.8 % | 0.8 % | 0.8 % |
| water | 86 % | 78 % | 64 % | 38 % |

The finely ground active ingredient is intimately mixed with the adjuvants, giving a suspension concentrate from which suspensions of any desired concentration can be obtained by dilution with water.

### EXAMPLE 14

### Gel formulation

The names of the formulation components are given according to the terminology of the registering authorities and their quantity is in grams per 100 g.

| Gel | | /100 g |
|---|---|---|
| active compound 6-(2-hydroxy-3-methylbenzylamino)purine | (2OH3MeBAP) | 1.0 g |
| butylhydroxytoluenum | (Nipanox BHT) | 0.2 g |
| butylparaben | (Nipabutyl) | 0.2 g |
| diethylene glycol monoethyl ether | (Transcutol P) | 10.0 g |
| silica colloidalis anhydrica | (Zeopharm 177) | 5.0 g |
| propylene glycol laurate | (Lauroglycol FCC) | 83.6 g |

The gel consistence may be additionally modified by addition of silica colloidalis anhydrica. It is expected that the transdermal Transcutol P/Lauroglycol FCC system will increase the efficiency of active compound. Silica colloidalis anhydrica will probably slow down the penetration of the active substance, which leads to a protracted effect.

## Claims

1. Substituted 6-(alkylbenzylamino)purine derivatives of general formula I 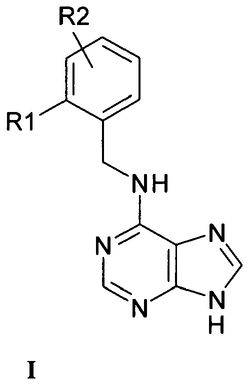 wherein
R1 denotes substituent independently selected from the group comprising hydroxyl, amino, nitro and thio group,
R2 denotes one to four alkyl groups, same or different,
and salts thereof with alkali metals, ammonium or amines,
in the form of racemates or optically active isomers, as well as their addition salts with acids
for use as cytokinin receptor antagonists.

2. Substituted 6-(alkylbenzylamino)purine derivatives according to claim 1, selected from the group comprising 6-(2-amino-3-methylbenzylamino)purine, 6-(2-amino-4-methylbenzylamino)purine, 6-(2-amino-5-methylbenzylamino)purine, 6-(2-amino-3-ethylbenzylamino)purine, 6-(2-amino-5-ethylbenzylamino)purine, 6-(2-amino-3-isopropylbenzylamino)purine, 6-(2-amino-5-isopropylbenzylamino)purine, 6-(2-hydroxy-3-methylbenzylamino)purine, 6-(2-hydroxy-4-methylbenzylamino)purine, 6-(2-hydroxy-5-methylbenzylamino)purine, 6-(2-hydroxy-6-methylbenzylamino)purine, 6-(2-hydroxy-3-ethylbenzylamino)purine, 6-(2-hydroxy-4-ethylbenzylamino)purine, 6-(2-hydroxy-5-ethylbenzylamino)purine, 6-(2-hydroxy-6-ethylbenzylamino)purine, 6-(2-hydroxy-3-isopropylbenzylamino)purine, 6-(2-hydroxy-5-isopropylbenzylamino)purine, 6-(2-nitro-3-methylbenzylamino)purine, 6-(2-nitro-4-methylbenzylamino)purine, 6-(2-thio-3-methylbenzylamino)purine, 6-(2-thio-5-methylbenzylamino)purine, 6-(2-thio-3-ethylbenzylamino)purine 6-(2-hydroxy-3,5-dimethylbenzylamino)purine, 6-(2-hydroxy-3,4-dimethylbenzylamino)purine, 6-(2-hydroxy-3,6-dimethylbenzylamino)purine, 6-(2-hydroxy-3,4,5-trimethylbenzylamino)purine, 6-(2-amino-3,5-dimethylbenzylamino)purine, 6-(2-amino-3,6-dimethylbenzylamino)purine, 6-(2-hydroxy-3,5-diethylbenzylamino)purine, 6-(2-hydroxy-3,6-diethylbenzylamino)purine,
and salts thereof with alkali metals, ammonium or amines,
in the form of racemates or optically active isomers, as well as their addition salts with acids
for use as cytokinin receptor antagonists.

3. Substituted 6-(alkylbenzylamino)purine derivatives according to claim 2, selected from the group containing 6-(2-hydroxy-3-methylbenzylamino)purine and 6-(2-hydroxy-5-methylbenzylamino)purine and salts thereof with alkali metals, ammonium or amines,in the form of racemates or optically active isomers, as well as their addition salts with acids for use as cytokinin receptor antagonists.

4. Use of substituted 6-(alkylbenzylamino)purine derivatives according to any of claims 1 to 3 as cytokinin receptor antagonists with morphogenetic effects leading to the increase of plant root system.

5. Use of substituted 6-(alkylbenzylamino)purine derivatives according to any of claims 1 to 3 as cytokinin receptor antagonists dispatching grain filling and increase of grain and fruit size of plants and fungi and for shortening of plant seed germination while leading to the increase of plant root system.

6. Use of substituted 6-(alkylbenzylamino)purine derivatives according to any of claims 1 to 3 as cytokinin receptor antagonists for increasing of yield and quality of agricultural products while leading to the increase of plant root system.

7. Use of substituted 6-(alkylbenzylamino)purine derivatives according to any of claims 1 to 3 as cytokinin receptor antagonists in the production of crops, in particular cereals, beets, pomes, drupes and soft fruits; leguminous plants, oil plants; cucumber plants, fibre plants, citrus fruits, vegetables or plants selected from the group comprising tobacco, nuts, eggplants, sugar cane, tea, vine grapes, hops, bananas and natural rubber and medicinal plants, as well as ornamentals, while leading to the increase of plant root system.

## Patentansprüche

1. Substituierte 6-(Alkylbenzylamino)purinderivate der allgemeinen Formel I 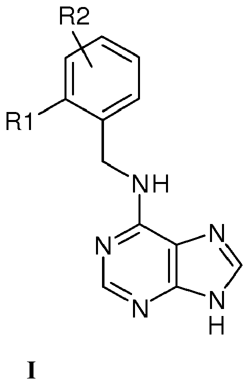 worin
R1 einen Substituent bedeutet, unabhängig ausgewählt aus einer Gruppe umfassend Hydroxyl, Amino, Nitro, und eine Thiogruppe,
R2 ein bis vier Alkylgruppen, identisch oder unterschiedlich, bedeutet,
und deren Salze mit Alkalimetallen, Ammonium oder Aminen,
in Form von Razematen oder optisch aktiven Isomeren, oder deren Additionssalzen mit Säuren zur Verwendung als Antagonisten von Cytokinin-Rezeptoren.

2. Substituierte 6-(Alkylbenzylamino)purinderivate nach dem Anspruch 1, ausgewählt aus einer Gruppe umfassend 6-(2-Amino-3-methylbenzylamino)purin, 6-(2-Amino-4-methylbenzylamino)purin, 6-(2-Amino-5-methylbenzylamino)purin, 6-(2-Amino-3-ethylbenzylamino)purin, 6-(2-Amino-5-ethylbenzylamino)purin, 6-(2-Amino-3-isopropylbenzylamino)purin, 6-(2-Amino-5-isopropylbenzylamino)purin, 6-(2-Hydroxy-3-methylbenzylamino)purin, 6-(2-Hydroxy-4-methylbenzylamino)purin, 6-(2-Hydroxy-5-methylbenzylamino)purin, 6-(2-Hydroxy-6-methylbenzylamino)purin, 6-(2-Hydroxy-3-ethylbenzylamino)purin, 6-(2-Hydroxy-4-ethylbenzylamino)purin, 6-(2-Hydroxy-5-ethylbenzylamino)purin, 6-(2-Hydroxy-6-ethylbenzylamino)purin, 6-(2-Hydroxy-3-isopropylbenzylamino)purin, 6-(2-Hydroxy-5-isopropylbenzylamino)purin, 6-(2-Nitro-3-methylbenzylamino)purin, 6-(2-Nitro-4-methylbenzylamino)purin, 6-(2-Thio-3-methylbenzylamino)purin, 6-(2-Thio-5-methylbenzylamino)purin, 6-(2-Thio-3-ethylbenzylamino)purin, 6-(2-Hydroxy-3,5-dimethylbenzylamino)purin, 6-(2-Hydroxy-3,4-dimethylbenzylamino)purin, 6-(2-Hydroxy-3,6-dimethylbenzylamino)purin, 6-(2-Hydroxy-3,4,5-trimethylbenzylamino)purin, 6-(2-Amino-3,5-dimethylbenzylamino)purin, 6-(2-Amino-3,6-dimethylbenzylamino)purin, 6-(2-Hydroxy-3,5-diethylbenzylamino)purin, 6-(2-Hydroxy-3,6-diethylbenzylamino)purin, und deren Salze mit Alkalimetallen, Ammonium oder Aminen,
in Form von Razematen oder optisch aktiven Isomeren, oder deren Additionssalzen mit Säuren zur Verwendung als Antagonisten von Cytokinin-Rezeptoren.

3. Substituierte 6-(Alkylbenzylamino)purinderivate nach dem Anspruch 2, ausgewählt aus einer Gruppe umfassend 6-(2-Hydroxy-3-methylbenzylamino)purin und 6-(2-Hydroxy-5-methylbenzylamino)purin und deren Salze mit Alkalimetallen, Ammonium oder Aminen, in Form von Razematen oder optisch aktiven Isomeren, oder deren Additionssalzen mit Säuren zur Verwendung als Antagonisten von Cytokinin-Rezeptoren.

4. Verwendung von substituierten 6-(Alkylbenzylamino)purinderivaten nach einem der Ansprüche 1 bis 3 als Antagonisten von Cytokinin-Rezeptoren mit morphogenen Wirkungen zur Ausdehnung des Wurzelsystems bei den Pflanzen.

5. Verwendung von substituierten 6-(Alkylbenzylamino)purinderivaten nach einem der Ansprüche 1 bis 3 als Antagonisten von Cytokinin-Rezeptoren zur Beschleunigung der Samenfüllung und Vergrösserung der Samengrösse und der Pflanzen-und Pilzenfrüchte und zur Verkürzung der Keimungsdauer bei Pflanzensamen und zur Förderung der Ausdehnung des Wurzelsystems bei den Pflanzen.

6. Verwendung von substituierten 6-(Alkylbenzylamino)purinderivaten nach einem der Ansprüche 1 bis 3 als Antagonisten von Cytokinin-Rezeptoren zur Ertrags- und Qualitätssteigerung bei Agrargütern und zur Förderung der Ausdehnung des Wurzelsystems bei den Pflanzen.

7. Verwendung von substituierten 6-(Alkylbenzylamino)purinderivaten nach einem der Ansprüche 1 bis 3 als Antagonisten von Cytokinin-Rezeptoren beim Anbau von Nutzpflanzen, insbesondere von Getreide, Rüben, Kernobst, Steinobst und Beerenobst; von Wickenpflanzen, Ölfrüchten, Kürbispflanzen, Faserpflanzen, Zitrusfrüchten, Gemüse, oder von Pflanzen, ausgewählt aus einer Gruppe umfassend Tabakpflanze, Nussbaum, Aubergine, Zuckerrohr, Teestaude, Weinrebe, Hopfen, Bananen und Naturkautschuk- und Heilpflanzen und Zierpflanzen, und zur Förderung der Ausdehnung des Wurzelsystems bei den Pflanzen.

## Revendications

1. Dérivés de 6-(alkylbenzylamino)purine substitués de formule générale **I** 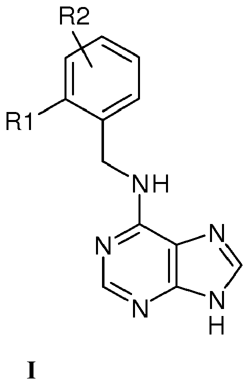 dans laquelle
R1 représente un substituant indépendamment choisi dans le groupe contentant des groupes hydroxyle, amino, nitro et thio,
R2 représente 1 à 4 groupes alkyles, identiques ou différents,
et leurs sels avec des métaux alcalins, ammonium ou amines,
sous forme de racémiques ou d'isomères optiquement actifs, ou de leurs sels d'addition avec les acides,
pour utilisation comme antagonistes du récepteur de la cytokinine.

2. Dérivés de 6-(alkylbenzylamino)purine substitués selon la revendication 1, choisis dans le groupe contenant 6-(2-amino-3-méthylbenzylamino)purine, 6-(2-amino-4-méthylbenzylamino)purine, 6-(2-amino-5-méthylbenzylamino)purine, 6-(2-amino-3-éthylbenzylamino)purine, 6-(2-amino-5-éthylbenzylamino)purine, 6-(2-amino-3-isopropylbenzylamino)purine, 6-(2-amino-5-isopropylbenzylamino)purine, 6-(2-hydroxy-3-méthylbenzylamino)purine, 6-(2-hydroxy-4-méthylbenzylamino)purine, 6-(2-hydroxy-5-méthylbenzylamino)purine, 6-(2-hydroxy-6-méthylbenzylamino)purine, 6-(2-hydroxy-3-éthylbenzylamino)purine, 6-(2-hydroxy-4-éthylbenzylamino)purine, 6-(2-hydroxy-5-éthylbenzylamino)purine, 6-(2-hydroxy-6-éthylbenzylamino)purine, 6-(2-hydroxy-3-isopropylbenzylamino)purine, 6-(2-hydroxy-5-isopropylbenzylamino)purine, 6-(2-nitro-3-méthylbenzylamino)purine, 6-(2-nitro-4-méthylbenzylamino)purine, 6-(2-thio-3-méthylbenzylamino)purine, 6-(2-thio-5-méthylbenzylamino)purine, 6-(2-thio-3-éthylbenzylamino)purine, 6-(2-hydroxy-3,5-diméthylbenzylamino)purine, 6-(2-hydroxy-3,4-diméthylbenzylamino)purine, 6-(2-hydroxy-3,6-diméthylbenzylamino)purine, 6-(2-hydroxy-3,4,5-triméthylbenzylamino)purine, 6-(2-amino-3,5-diméthylbenzylamino)purine, 6-(2-amino-3,6-diméthylbenzylamino)purine, 6-(2-hydroxy-3,5-diéthylbenzylamino)purine, 6-(2-hydroxy-3,6-diéthylbenzylamino)purine, et leurs sels avec des métaux alcalins, ammonium ou amines, sous forme de racémiques ou d'isomères optiquement actifs, ou de leurs sels d'addition avec les acides,
pour utilisation comme antagonistes du récepteur de la cytokinine.

3. Dérivés de 6-(alkylbenzylamino)purine substitués selon la revendication 2, choisis dans le groupe contenant 6-(2-hydroxy-3-méthylbenzylamino)purine et 6-(2-hydroxy-5-méthylbenzylamino)purine et leurs sels avec des métaux alcalins, ammonium ou amines, sous forme de racémiques ou d'isomères optiquement actifs, ou de leurs sels d'addition avec les acides, pour utilisation comme antagonistes du récepteur de la cytokinine.

4. Utilisation de dérivés de 6-(alkylbenzylamino)purine substitués selon l'une quelconque revendication 1 à 3 comme antagonistes du récepteur de la cytokinine avec effets morphogènes menant à la croissance du système racinaire des plantes.

5. Utilisation de dérivés de 6-(alkylbenzylamino)purine substitués selon l'une quelconque revendication 1 à 3 comme antagonistes du récepteur de la cytokinine pour accélérer le gonflage des graines et la croissance de la taille de la graine et des fruits des plantes et champignons et pour réduire la durée de germination des graines des plantes et menant à la croissance du système racinaire des plantes.

6. Utilisation de dérivés de 6-(alkylbenzylamino)purine substitués selon l'une quelconque revendication 1 à 3 comme antagonistes du récepteur de la cytokinine pour augmenter le rendement et la qualité des produits agricoles et menant à la croissance du système racinaire des plantes.

7. Utilisation de dérivés de 6-(alkylbenzylamino)purine substitués selon l'une quelconque revendication 1 à 3 comme antagonistes du récepteur de la cytokinine dans la production des plantes utilitaires, notamment des céréales, betteraves, fruits à pépins, fruits à noyau et plantes baccifères; plantes légumineuses, oléagineuses, cucurbitacées, plantes à filer, agrumes, légumes ou des plantes choisies dans le groupe comprenant le tabac, noyer, aubergine, canne à sucre, théier, vigne, houblon, banane et les plantes naturelles à caoutchouc et plantes médicinales et plantes décoratives, et menant à la croissance du système racinaire des plantes.
